(19) Europäisches Patentamt
European Patent Office
Office européen des brevets

(11) Veröffentlichungsnummer: **0 022 538**
**B1**

(12) **EUROPÄISCHE PATENTSCHRIFT**

(45) Veröffentlichungstag der Patentschrift: **15.09.82**

(51) Int. Cl.$^3$: **C 07 C 143/70**

(21) Anmeldenummer: **80103871.2**

(22) Anmeldetag: **08.07.80**

(54) **Verfahren zur Herstellung von Naphthalinsulfochlorid.**

(30) Priorität: **17.07.79 DE 2928744**

(43) Veröffentlichungstag der Anmeldung: **21.01.81 Patentblatt 81/3**

(45) Bekanntmachung des Hinweises auf die Patenterteilung: **15.09.82 Patentblatt 82/37**

(84) Benannte Vertragsstaaten: **BE CH DE FR GB IT LI**

(56) Entgegenhaltungen:
**DE-A-1 963 383**
**HELVETICA CHIMICA ACTA, Band 42, Nr. 5, 1959 H. H. Bosshard et al., »Eine Methode zur katalysierten Herstellung von Carbonsäure- und Sulfosäurechloriden mit Thionylchlorid«, Seiten 1653 bis 1658.**
**SYNTHESIS, Band 1974, A Barco et al., »A New Preparation of Sulfonyl Chlorides via Pyridinium Sulfonates«, Seite 877.**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Rauchschwalbe, Günter, Dr., Hofstrasse 28, D-5000 Köln 80 (DE)**
Erfinder: **Mannes, Karl, Dr., Kurt-Schumacher-Ring 119, D-5090 Leverkusen 1 (DE)**
Erfinder: **Mayer, Dietmar, Dr., Hamberger Strasse 51, D-5090 Leverkusen 3 (DE)**

**EP 0 022 538 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Naphthalinsulfochlorid

Die Erfindung betrifft ein Verfahren zur Herstellung von Naphthalinsulfochloriden durch Umsetzung der Alkalimetall- oder Ammoniumsalze der entsprechenden Naphthalinsulfonsäuren mit Thionylchlorid in Gegenwart von katalytisch wirksamen Substanzen und gegebenenfalls in Gegenwart von inerten Lösungs- oder Verdünnungsmitteln.

Es ist bekannt, Naphthalinsulfochloride aus den entsprechenden Sulfonsäuren oder deren Alkalimetallsalze mit Thionylchlorid in Gegenwart von Dialkylformamiden, bevorzugt Dimethylformamid, als Katalysator und gegebenenfalls in Gegenwart von inerten Lösungsmitteln darzustellen (Helv. Chim. Acta 42, 1654 [1959]).

Obwohl sich nach diesem Verfahren Naphthalinsulfochloride in guten Ausbeuten herstellen lassen, bringt die Verwendung von Dimethylformamid schwerwiegende Nachteile mit sich. So hat sich herausgestellt, daß aus Dimethylformamid und Thionylchlorid Dimethylcarbamoylchlorid entstehen kann (vgl. C. A. 75, 48340 m), das bei Mäusen eine starke cancerogene Wirkung zeigt (vgl. C. A. 77, 97540 b).

Es ist weiterhin bekannt, daß Pyridiniumsalze von Sulfonsäuren mit Thionylchlorid in guten Ausbeuten Sulfochloride bilden (Synthesis 1974, 877). Gemäß dieser Literaturstelle wird zunächst das Pyridiniumsalz der Sulfonsäuren mit überschüssiger Base hergestellt und isoliert und anschließend in kristalliner Form mit Thionylchlorid umgesetzt.

Das Verfahren hat jedoch den Nachteil, daß zunächst das Pyridiniumsalz der Sulfonsäure isoliert und gereinigt werden muß und zur Herstellung des Pyridiniumsalzes ein Überschuß an Pyridin erforderlich ist. Dadurch wird das Verfahren technisch aufwendig und weniger wirtschaftlich.

Es wurde nun ein Verfahren zur Herstellung von Naphthalinsulfochloriden durch Umsetzung der Alkalimetall- oder Ammoniumsalze der Naphthalinsulfonsäuren mit Thionylchlorid in Gegenwart von katalytisch wirksamen Substanzen und gegebenenfalls in Gegenwart von inerten Lösungs- oder Verdünnungsmitteln gefunden, das dadurch gekennzeichnet ist, daß man die Umsetzung in Gegenwart von 0,5—10 Gew.-%, bezogen auf die Alkalimetall- oder Ammoniumsalze der Naphthalinsulfonsäuren von gegebenenfalls substituierten Pyridinen, tert. aliphatischen Aminen, sekundären Amidinen und/oder quartären Ammoniumsalzen durchführt.

Als gegebenenfalls substituierte Pyridine können solche der allgemeinen Formel (I)

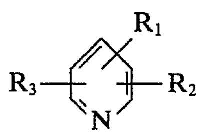 (I)

in der

$R_1$, $R_2$ und $R_3$ gleich oder verschieden sind und für Wasserstoff, Halogen, eine Hydroxy-, Sulfo- oder Cyanogruppe oder einen Alkyl-, Aryl-, Aralkyl-, Dialkylamino- oder N-Pyridinorest oder wobei zwei der Reste $R_1$ bis $R_3$, wenn sie benachbart sind, für einen Benzorest stehen,

in das erfindungsgemäße Verfahren eingesetzt werden.

Als Halogene seien genannt: Fluor, Chlor, Brom, Jod, bevorzugt Chlor, Brom.

Als Reste $R_1$, $R_2$ und $R_3$ kommen beispielsweise in Betracht: Alkylreste mit bis zu 10 C-Atomen, bevorzugt bis zu 5 C-Atomen, wie Methyl, Ethyl, n-Propyl, iso-Propyl, n-Butyl, iso-Butyl, tert.-Butyl, n-Pentyl, iso-Pentyl, Hexyl, Octyl, Decyl, Cyclohexyl, Decahydronaphthyl, bevorzugt Methyl, Ethyl, n-Propyl; Arylreste mit bis zu 10 C-Atomen, bevorzugt bis zu 6 C-Atomen, wie Phenyl, Tolyl, Naphthyl, bevorzugt Phenyl, Tolyl; Aralkylreste mit bis zu 14 C-Atomen, bevorzugt bis zu 8 C-Atomen, wie Benzyl, Phenylethyl, Phenylpropyl, Phenylbutyl, Dihydronaphthyl, Tetrahydronaphthyl, Naphthylmethyl, Naphthylethyl, bevorzugt Benzyl, Phenylethyl;

Dialkylaminoreste mit bis zu 10 C-Atomen, bevorzugt bis zu 6 C-Atomen, wie Dimethyl-, Diethyl-, Dipropyl-, Dibutyl-, Diamyl-, Ethylhexyl-, Methylcyclohexylamino, Pyrrolidino, Piperidino, Morpholino, bevorzugt Dimethylamino, Pyrrolidino, Piperidino, Morpholino; N-Pyridino-Reste mit bis zu 12 C-Atomen, bevorzugt bis zu 8 C-Atomen wie N-Pyridino, N-Picolino, N-Lutidino, N-Collidino, N-(Methylethylpyridino), N-Chinolino, N-Isochinolino, bevorzugt N-Pyridino, N-Picolino, N-Lutidino.

Als Benzoreste seien solche mit bis zu 10 C-Atomen, bevorzugt bis zu 6 C-Atomen, genannt, wie Benzo, Naphtho, bevorzugt Benzo.

Zum Beispiel seien als gegebenenfalls substituierte Pyridine genannt: Pyridin, Picolin, Lutidin, Collidin, Methylethylpyridin, (N-Pyridino)-pyridinchlorid, Chlorpyridin, Cyanopyridin, Hydroxypyridin, Pyridinsulfosäure, Dimethylaminopyridin, Morpholino-, Pyrrolidino-, Piperidinopyridin, Chinolin, Isochinolin, bevorzugt Pyridin, Picolin, Lutidin, 4-Cyanopyridin, 4-Dimethylaminopyridin, Isochinolin.

Selbstverständlich können auch Additionsverbindungen aus gegebenenfalls substituiertem Pyridin und Thionylchlorid in das erfindungsgemäße Verfahren eingesetzt werden.

Als tertiäre aliphatische Amine können solche der allgemeinen Formel (II)

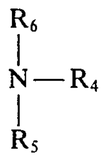

(II)

in der

$R_4$, $R_5$ und $R_6$ gleich oder verschieden sind und für einen Alkyl-, Cycloalkyl-, N-Alkenylaldimino-, N-Alkenylketimino- oder einen N,N-Dialkylaminoalkylenrest stehen oder zwei beziehungsweise drei der Reste zusammen ein gegebenenfalls ein oder mehrere Heteroatome enthaltendes mono- beziehungsweise bicyclisches Ringsystem mit bis zu 10 C-Atomen, bevorzugt bis zu 8 C-Atomen, bilden,

in das erfindungsgemäße Verfahren eingesetzt werden.

Als Reste $R_4$, $R_5$ und $R_6$ kommen beispielsweise in Betracht: Alkylreste mit bis zu 6 C-Atomen, bevorzugt bis zu 3 C-Atomen, wie Methyl, Ethyl, Propyl, n-Butyl, Isobutyl, tert.-Butyl, Pentyl, Hexyl, bevorzugt Methyl, Ethyl;

Cycloalkylreste mit bis zu 10 C-Atomen, bevorzugt bis zu 6 C-Atomen, wie Cyclopentyl, Cyclohexyl, Methylcyclohexyl, Cyclooctyl, Cyclodecyl, Decahydronaphthyl, bevorzugt Cyclohexyl;

N-Alkenylaldiminoreste mit bis zu 15 C-Atomen, bevorzugt bis zu 12 C-Atomen, wie 2-Aza-buta-1,3-dien-1,4-diyl, 2-Aza-pent-1-en-1,5-diyl, bevorzugt 2-Aza-buta-1,3-dien-1,4-diyl;

N-Alkenylketiminoreste mit bis zu 15 C-Atomen, bevorzugt bis zu 12 C-Atomen, wie 1-Methyl-2-aza-buta-1,3-dien-1,4-diyl, 1-Butyl-2-aza-pent-1-en-1,5-diyl, bevorzugt 1-Methyl-2-aza-buta-1,3-dien-1,4-diyl;

N,N-Dialkylaminoalkylenreste mit bis zu 9 C-Atomen, bevorzugt bis zu 4 C-Atomen, wie Dimethylaminoethyl, Diethylaminoethyl, Dimethylaminopropyl, Dimethylaminobutyl, bevorzugt Dimethylaminoethyl.

Als gegebenenfalls ein oder mehrere Heteroatome, wie Sauerstoff, Schwefel oder Stickstoff, enthaltende mono- oder bicyclische Ringsysteme seien z. B. genannt: Pyrrolidin, Piperidin, Morpholin, Diazabicyclooctan, Chinuklidin, 4-Thiomorpholin, bevorzugt Pyrrolidin, Piperidin, Morpholin.

Zum Beispiel seien als tertiäre aliphatische Amine genannt: Trimethylamin, Triethylamin, N-Methylpyrrolidin, N-Methylmorpholin, N-Methylpiperidin, Tetramethylethylendiamin, Bis-(2-dimethylaminoethyl)-methylamin, 1,4-Dimethylpiperazin, 1,4-Diazabicyclooctan, Chinuklidin, 1,4,5,6-Tetrahydro-1,2-dimethylpyrimidin, 1-Methylimidazol, 1,2-Dimethylimidazol, 1,5-Diazabicyclo[4.3.0]-non-5-en, 1,8-Diaza-bicyclo-[5.4.0]-undec-7-en, bevorzugt Trimethylamin, Triethylamin, N-Methylpyrrolidin.

Die tert. aliphatischen Amine können selbstverständlich auch in Form ihrer Salze wie Hydrohalogenide, Sulfate, Phosphate in das erfindungsgemäße Verfahren eingesetzt werden.

Als quartäre Ammoniumsalze können solche der allgemeinen Formel (III)

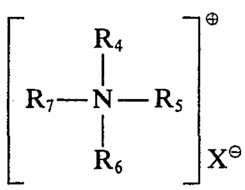

(III)

in der

$R^4$, $R^5$, $R^6$ und $R^7$ gleich oder verschieden sind und für einen Alkyl-, Cycloalkyl-, N-Alkenylaldimino-, N-Alkenylketimino oder einen N,N-Dialkylaminoalkylenrest stehen oder zwei beziehungsweise drei der Reste zusammen ein gegebenenfalls ein oder mehrere Heteroatome enthaltendes mono- beziehungsweise bicyclisches Ringsystem mit bis zu 10 C-Atomen, bevorzugt bis zu 8 C-Atomen, bilden und

$X^{\ominus}$ für Chlorid, Bromid, Jodid, Sulfat, Phosphat oder Hydrogensulfat steht,

in das erfindungsgemäße Verfahren eingesetzt werden.

Zum Beispiel seien als quartäre Ammoniumsalze genannt: Tetramethylammoniumchlorid, Tetraethylammoniumchlorid, N,N-Dimethylpyrrolidiniumbromid, Hexaethylethylendiammoniumchlorid, bevorzugt Tetramethylammoniumchlorid, Tetraethylammoniumchlorid.

Als sekundäre Amidine können solche der allgemeinen Formel (IV)

$R_8-NH-CH=N-R_9$ (IV)

in der

$R_8$ und $R_9$ gleich oder verschieden sind und für einen Alkyl- oder Cycloalkylrest stehen oder zusammen ein mono- oder bicyclisches Ringsystem mit bis zu 15 C-Atomen, bevorzugt bis zu 9 C-Atomen, bilden,

in das erfindungsgemäße Verfahren eingesetzt werden.

Als Reste $R_8$ und $R_9$ kommen beispielsweise in Betracht: Alkylreste mit bis zu 10 C-Atomen, bevorzugt bis zu 8 C-Atomen, wie Methyl, Ethyl, n-Propyl, iso-Propyl, n-Butyl, iso-Butyl, tert.-Butyl, n-Pentyl, iso-Pentyl, Hexyl, Octyl, bevorzugt Methyl, Ethyl, n-Propyl; Cycloalkylreste mit bis zu 10 C-Atomen, bevorzugt bis zu 8 C-Atomen, wie Cyclohexyl, Methylcyclohexyl, Cyclooxyl, Cyclodecyl, bevorzugt Cyclohexyl.

Als mono- oder bicyclische Ringsysteme gemäß der allgemeinen Formel (IV) seien genannt: Imidazol, Hexahydrobenzimidazol, Imidazolin, bevorzugt Imidazol.

Zum Beispiel seien als sekundäre Amidine genannt: N,N'-Dimethylformamidin, N-Ethyl-N'-cyclohexyl-formamidin, Imidazol, Imidazolin, Hexahydrobenzimidazol, bevorzugt Imidazol.

Die gegebenenfalls substituierten Pyridine, tertiären aliphatischen Amine, sekundären Amidine und/oder quartären Ammoniumsalze werden in Mengen von 0,5 bis 10 Gew.-%, bevorzugt 2 bis 5 Gew.-%, besonders bevorzugt 3 bis 4 Gew.-%, bezogen auf die eingesetzten Alkalimetall- oder Ammoniumsalze der Naphthalinsulfonsäure, in das erfindungsgemäße Verfahren eingesetzt.

Als Alkalimetallsalze der Naphthalinsulfonsäuren kommen die Lithium-, Natrium-, Kalium- und/oder Rubidiumsalze, vorzugsweise das Natrium- oder Kaliumsalze, in Betracht.

Als Naphthalinsulfonsäuren, die in Form ihrer Alkalimetallsalze oder Ammoniumsalze, in das erfindungsgemäße Verfahren eingesetzt werden können, seien z. B. genannt: 1-Naphthalinsulfosäure, 2-Naphthalinsulfosäure, 1,4-, 1,5-, 1,6-, 2,6-, 2,7-Naphthalindisulfosäure, Naphthalin-1,3,6-trisulfosäure, -1,3,5-trisulfosäure, -1,3,7-trisulfosäure, bevorzugt 1-Naphthalinsulfosäure, 2-Naphthalinsulfosäure, 1,5-Naphthalindisulfosäure.

Nach dem erfindungsgemäßen Verfahren werden die Alkalimetall- oder Ammoniumsalze der Naphthalinsulfonsäuren im allgemeinen mit etwa der äquivalenten Menge, bevorzugt mit 1,1 bis 1,5 Moläquivalenten, besonders bevorzugt mit 1,2 bis 1,3 Moläquivalenten, Thionylchlorid umgesetzt.

Es ist jedoch auch möglich, die Umsetzung der Naphthalinsulfonsäuresalze mit einem größeren Überschuß an Thionylchlorid durchzuführen, besonders dann, wenn das Thionylchlorid die Rolle des aufschlämmenden flüssigen Reaktionsmediums einnehmen soll.

Dann werden z. B. etwa 1,5 bis etwa 5 Mol, bevorzugt 2 bis 3 Mol, Thionylchlorid pro Mol Sulfonat eingesetzt. Das überschüssige Thionylchlorid kann nach Ende der Umsetzung wiedergewonnen und erneut in die Reaktion eingesetzt werden.

Man kann die erfindungsgemäße Umsetzung auch in Gegenwart von inerten Lösungs- oder Verdünnungsmitteln durchführen, wobei dann die oben angegebenen etwa äquimolaren Mengen an Thionylchlorid genügen.

Als Lösungs- oder Verdünnungsmittel eignen sich vor allem solche Verbindungen, die unter den Reaktionsbedingungen inert sind und das entstehende Naphthalinsulfochlorid wenigstens teilweise zu lösen vermögen.

Zum Beispiel seien genannt: Aliphatische oder aromatische Kohlenwasserstoffe mit bis zu 15 C-Atomen, bevorzugt bis zu 8 C-Atomen, wie Hexan, Cyclohexan, Toluol, Xylol, Octan, Dekalin, Cumol, Mesitylen, Tetralin, bevorzugt Toluol sowie halogenierte aliphatische oder aromatische Kohlenwasserstoffe mit bis zu 15 C-Atomen, bevorzugt bis zu 8 C-Atomen, wie Methylenchlorid, Chloroform, Dichlorethan, Trichlorethylen, Tetrachlorethan, 1,1,2,3,3-Pentachlorpropan, Hexachlorcyclopentadien, Octachlorcyclopentan, Chlorbenzol, Di-, Trichlorbenzol, Chlortoluol, Chlorxylol, bevorzugt Chlorbenzol.

Die Lösungs- oder Verdünnungsmittel können einzeln oder im Gemisch untereinander eingesetzt werden.

Im allgemeinen werden die Lösungs- oder Verdünnungsmittel in Mengen von etwa $^1/_2$ bis etwa 5 Liter pro Mol eingesetztes Sulfonat verwendet.

Das erfindungsgemäße Verfahren kann bei Temperaturen im Bereich von etwa 20 bis etwa 140° C, bevorzugt bei 50 bis 130° C, besonders bevorzugt bei 80 bis 100° C, durchgeführt werden.

Obwohl das erfindungsgemäße Verfahren bevorzugt bei Normaldruck durchgeführt wird, ist es auch möglich, bei erhöhtem Druck (bis etwa 50 bar) zu arbeiten.

Nach dem erfindungsgemäßen Verfahren kann das Thionylchlorid vorgelegt und das Sulfonat zugegeben oder umgekehrt Sulfonat vorgelegt und das Thionylchlorid zugegeben werden.

Die gegebenenfalls substituierten Pyridine, tert. aliphatischen Amine, sekundären Amidine und/oder quartären Ammoniumsalze können vor, während oder nach der Zugabe der zweiten Hauptkomponente dem Reaktionsgemisch zugesetzt werden.

Wird in einem inerten Lösungs- oder Verdünnungsmittel gearbeitet, ist es vorteilhaft, wenn man die

Alkalimetall- oder Ammoniumsalze der Naphthalinsulfonsäuren mit den inerten Lösungs- oder Verdünnungsmitteln aufschlämmt und dann das Thionylchlorid sowie die gegebenenfalls substituierten Pyridine, tert. aliphatischen Amine, sekundären Amidine und/oder quartären Ammoniumsalze hinzugibt.

Nach Beendigung der Umsetzung wird das inerte Lösungs- oder Verdünnungsmittel und/oder überschüssiges Thionylchlorid abdestilliert, wobei sowohl das inerte Lösungs- oder Verdünnungsmittel als auch das Thionylchlorid wieder in die Reaktion eingesetzt werden kann.

Das verbleibende Rohprodukt kann als solches direkt, z. B. zur Herstellung von Azofarbstoffen, wie in der britischen Patentschrift 634 488 oder der Schweizer Patentschrift 261 840 beschrieben, verwendet werden oder es kann, wenn die Weiterverarbeitung es erfordert, durch Destillation im Vakuum oder Umkristallisation mit geeigneten Lösungsmitteln gereinigt werden.

Das erfindungsgemäße Verfahren kann sowohl kontinuierlich als auch diskontinuierlich durchgeführt werden.

Die nach dem erfindungsgemäßen Verfahren erzielten Ausbeuten an Naphthalinsulfochloriden sind hoch, sie liegen zwischen etwa 80 und 100% der Theorie.

Es ist außerordentlich überraschend, daß das erfindungsgemäße Verfahren in einer glatt verlaufenden Reaktion solch gute Ausbeuten an Naphthalinsulfochloriden liefert. Aus Helv. Chim. Acta 42, 1654 (1959) ist nämlich bekannt, daß die Umsetzung mit Thionylchlorid in Gegenwart von Pyridin als Katalysator auf Carbonsäuren beschränkt ist.

Die nachfolgenden Beispiele sollen das erfindungsgemäße Verfahren verdeutlichen.

Beispiel 1

104 g Natrium-2-naphthalinsulfonat werden langsam zu einer Mischung aus 100 ml Thionylchlorid und 3,0 g Pyridin gegeben, die im Ölbad auf 40° C erwärmt wurde.

Bei einer Ölbadtemperatur von 100° C wird dann noch 1,5 Stunden am Rückfluß zum Sieden erwärmt, bis die Gasentwicklung beendet ist, und dann noch 2 Stunden nachgerührt.

Überschüssiges Thionylchlorid wird abdestilliert. Man erhält 130,3 g Rohprodukt, das 78,6% 2-Naphthalinsulfochlorid enthält (Anilintitration), das entspricht 99,9% Ausbeute, d. h. quantitative Ausbeute im Rahmen der analytischen Fehlergrenzen.

Weitere Beispiele bei entsprechender experimenteller Durchführung seien in Form einer Tabelle angegeben.

Tabelle

| Beisp. | Einsatz an Natrium-2-naphthalin-sulfonat [Mol] | Katalysator | Menge [Gew.-%] | Ausbeute an Naphthalin-2-sulfochlorid [%] |
|---|---|---|---|---|
| 2 | 2,3 | Pyridin | 3 | 100 |
| 3 | 0,46 | 4-Picolin | 3 | 98,7 |
| 4 | 0,46 | 4-Cyanopyridin | 3 | 100 |
| 5 | 0,47 | 2-Picolin | 3 | 91,3 |
| 6 | 0,46 | 4-(N,N-Dimethyl-amino)-pyridin | 3 | 99,6 |
| 7 | 0,50 | Isochinolin | 6 | 100 |
| 8 | 0,46 | Trimethylamin (als Hydrochlorid) | 3 | 93,5 |
| 9 | 0,47 | Triethylamin | 3 | 93,3 |
| 10 | 0,46 | N-Methylpyrrolidin | 3 | 100 |
| 11 | 0,46 | Imidazol | 3 | 96,9 |
| 12 | 0,46 | Tetraethylammoniumchlorid | 3 | 82,0 |
| 13 | 0,46 | Pyridin | 2 | 89,6 |
| 14 | 0,46 | Pyridin | 4 | 99,3 |
| 15 | 0,46 | Pyridin | 10 | 100 |

Beispiel 16

1 Mol Natrium-2-naphthalinsulfonat und 7,0 g Pyridin werden in 600 ml Chlorbenzol suspendiert und auf 40°C erwärmt. Unter Rühren tropft man 143 g (1,2 Mol) Thionylchlorid zu, erwärmt noch 6 Stunden lang auf 100°C und destilliert Chlorbenzol und restliches Thionylchlorid im Vakuum ab.

Die Ausbeute an Naphthalinsulfochlorid beträgt 99%.

Beispiel 17

115,1 g (0,5 Mol) Natrium-1-naphthalinsulfonat werden wie in Beispiel 1 umgesetzt; man gibt jedoch 6,0 g Pyridin zu und rührt insgesamt 3 Stunden. Die Ausbeute betrug 146,4 g Rohprodukt, das 76,8% Naphthalin-1-sulfochlorid enthielt (99,1% Ausbeute).

Beispiel 18

116,0 g (0,5 Mol) gereinigtes Naphthalin-1,5-disulfonsäuredinatriumsalz, 6 g Pyridin und 300 g $SOCl_2$ wurden entsprechend Beispiel 1 miteinander umgesetzt. Man erhielt Naphthalin-1,5-disulfochlorid, das noch ca. 28% Kochsalz enthielt. Die Ausbeute betrug nach der Anilintitrationsmethode 90%.

Beispiel 19

Man arbeitet wie in Beispiel 14 beschrieben, verwendet jedoch das Kaliumsalz der Naphthalin-2-sulfosäure. Die Ausbeute war quantitativ.

**Patentansprüche**

1. Verfahren zur Herstellung von Naphthalinsulfochloriden durch Umsetzung der Alkalimetall- oder Ammoniumsalze der Naphthalinsulfonsäuren mit Thionylchlorid in Gegenwart von katalytisch wirksamen Substanzen und gegebenenfalls in Gegenwart von inerten Lösungs- oder Verdünnungsmitteln, dadurch gekennzeichnet, daß man die Umsetzung in Gegenwart von 0,5—10 Gew.-%, bezogen auf die Alkalimetall- oder Ammoniumsalze der Naphthalinsulfonsäuren von gegebenenfalls substituierten Pyridinen, tert. aliphatischen Aminen, sekundären Amidinen und/oder quartären Ammoniumsalzen durchführt.

2. Verfahren nach Anspruch 1, dadurch gekennzeichnet, daß man als gegebenenfalls substituierte Pyridine solche der allgemeinen Formel (I)

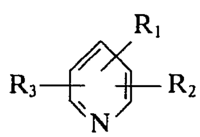

(I)

einsetzt, in der

$R_1$, $R_2$ und $R_3$ gleich oder verschieden sind und für Wasserstoff, Halogen, eine Hydroxy-, Sulfo- oder Cyanogruppe oder einen Alkyl-, Aryl-, Aralkyl-, Dialkylamino- oder N-Pyridinorest oder wobei zwei der Reste $R_1$ bis $R_3$, wenn sie benachbart sind, für einen Benzorest stehen.

3. Verfahren nach Ansprüchen 1 und 2, dadurch gekennzeichnet, daß man Pyridin, Picolin, Lutidin, 4-Dimethylaminopyridin, 4-Cyanopyridin, Isochinolin einsetzt.

4. Verfahren nach Anspruch 1, dadurch gekennzeichnet, daß man als tertiäre aliphatische Amine solche der allgemeinen Formel (II)

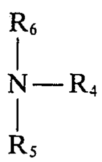

(II)

einsetzt, in der

$R_4$, $R_5$ und $R_6$ gleich oder verschieden sind und für einen Alkyl-, Cycloalkyl-, N-Alkenylaldimino-, N-Alkenylketimino- oder einen N,N-Dialkylaminoalkylenrest stehen oder zwei beziehungsweise drei der Reste zusammen ein gegebenenfalls ein oder mehrere Heteroatome enthaltendes mono- beziehungsweise bicyclisches Ringsystem mit bis zu 10 C-Atomen bilden.

5. Verfahren nach Ansprüchen 1 und 4, dadurch gekennzeichnet, daß man Trimethylamin, Triethylamin, N-Methylpyrrolidin einsetzt.

6. Verfahren nach Anspruch 1, dadurch gekennzeichnet, daß man als quartäre Ammoniumsalze solche der allgemeinen Formel (III)

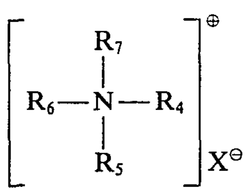

(III)

einsetzt, in der

$R^4$, $R^5$, $R^6$ und $R^7$ gleich oder verschieden sind und für einen Alkyl-, Cycloalkyl-, N-Alkenylaldimino-, N-Alkenylketimino oder einen N,N-Dialkylaminoalkylenrest stehen oder zwei beziehungsweise drei der Reste zusammen ein gegebenenfalls ein oder mehrere Heteroatome enthaltendes mono- beziehungsweise bicyclisches Ringsystem mit bis zu 10 C-Atomen, bevorzugt bis zu 8 C-Atomen, bilden und

$X^{\ominus}$ für Chlorid, Bromid, Jodid, Sulfat, Phosphat oder Hydrogensulfat steht.

7. Verfahren nach Ansprüchen 1 und 6, dadurch gekennzeichnet, daß man Tetramethylammoniumchlorid, Tetraethylammoniumchlorid einsetzt.

8. Verfahren nach Anspruch 1, dadurch gekennzeichnet, daß man als sekundäre Amidine solche der allgemeinen Formel (IV)

$$R_8-NH-CH=N-R_9 \qquad (IV)$$

einsetzt, in der

$R_8$ und $R_9$ gleich oder verschieden sind und für einen Alkyl- oder Cycloalkylrest stehen oder zusammen ein mono- oder bicyclisches Ringsystem mit bis zu 15 C-Atomen bilden.

9. Verfahren nach Ansprüchen 1 und 8, dadurch gekennzeichnet, daß man Imidazol einsetzt.

**Claims**

1. Process for the preparation of naphthalenesulphonyl chlorides by reacting the alkali metal salts or ammonium salts of naphthalenesulphonic acids with thionyl chloride in the presence of catalytically active substances and if appropriate in the presence of inert solvents or diluents, characterised in that the reaction is carried out in the presence of 0.5—10% by weight, relative to the alkali metal salts or ammonium salts of naphthalenesulphonic acids, of optionally substituted pyridines, tertiary aliphatic amines, secondary amidines and/or quaternary ammonium salts.

2. Process according to Claim 1, characterised in that optionally substituted pyridines which are employed are those of the general formula (I)

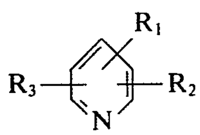

(I)

in which

$R_1$, $R_2$ and $R_3$ are identical or different and represent hydrogen, halogen, a hydroxyl, sulpho or cyano group or an alkyl, aryl, aralkyl, dialkylamino or N-pyridino radical, or wherein two of the radicals $R_1$ to $R_3$, if they are adjacent, represent a benzo radical.

3. Process according to Claims 1 and 2, characterised in that pyridine, picoline, lutidine, 4-dimethylaminopyridine, 4-cyanopyridine or isoquinoline is employed.

4. Process according to Claim 1, characterised in that the tertiary aliphatic amines employed are those of the general formula (II)

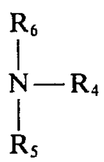

(II)

in which

$R_4$, $R_5$ and $R_6$ are identical or different and represent an alkyl, cycloalkyl, N-alkenylaldimino, N-alkenylketimino or N,N-dialkylaminoalkylene radical, or two or three of the radicals together form a monocyclic or bicyclic ring system which has up to 10 C atoms and optionally contains one or more hetero-atoms.

5. Process according to Claims 1 and 4, characterised in that trimethylamine, triethylamine or N-methylpyrrolidine is employed.

6. Process according to Claim 1, characterised in that the quaternary ammonium salts employed are those of the general formula (III)

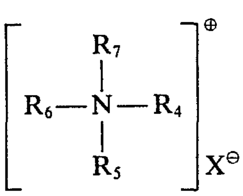

(III)

in which

$R^4$, $R^5$, $R^6$ and $R^7$ are identical or different and represent an alkyl, cycloalkyl, N-alkenylaldimino, N-alkenylketimino or N,N-dialkylaminoalkylene radical, or two or three of the radicals together form a monocyclic or bicyclic ring system which has up to 10 C atoms, preferably up to 8 C atoms, and optionally contains one or more hetero-atoms, and

$X^{\ominus}$ represents chloride, bromide, iodide, sulphate, phosphate or bisulphate.

7. Process according to Claims 1 and 6, characterised in that tetramethylammonium chloride or tetraethylammonium chloride is employed.

8. Process according to Claim 1, characterised in that the secondary amidines employed are those of the general formula (IV)

$$R_8-NH-CH=N-R_9 \qquad (IV)$$

in which

$R_8$ and $R_9$ are identical or differenz and represent an alkyl or cycloalkyl radical or together form a monocyclic or bicyclic ring system with up to 15 C atoms.

9. Process according to Claims 1 and 8, characterised in that imidazole is employed.

**Revendications**

1. Procédé de préparation de naphtalènesulfochlorures par réaction des sels de métaux alcalins ou d'ammonium des acides naphtalène-sulfoniques avec du chlorure de thionyle en présence de substances à activité catalytique et éventuellement en présence de solvants ou de diluants inertes, caractérisé en ce qu'on effectue la réaction en présence de 0,5 à 10% en poids (calculé sur les sels de métaux alcalins ou d'ammonium des acides naphtalène-sulfoniques) de pyridines éventuellement substituées, d'amines tertiaires aliphatiques, d'amidines secondaires et/ou de sels d'ammonium quaternaire.

2. Procédé suivant la revendication 1, caractérisé en ce que, comme pyridines éventuellement substituées, on utilise celles répondant à la formule générale (I):

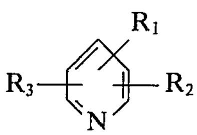

(I)

dans laquelle

$R_1$, $R_2$ et $R_3$ sont identiques ou différents et représentent chacun un atome d'hydrogène, un atome d'halogène, un groupe hydroxy, un groupe sulfo ou un groupe cyano, ou encore un groupe alkyle, aryle, aralkyle, dialkylamino ou N-pyridino, ou deux des radicaux $R_1$ à $R_3$ représentent, lorsqu'ils sont voisins, un groupe benzo.

3. Procédé suivant les revendications 1 et 2, caractérisé en ce qu'on utilise la pyridine, la picoline, la lutidine, la 4-diméthylaminopyridine, la 4-cyanopyridine ou l'isoquinoléine.

4. Procédé suivant la revendication 1, caractérisé en ce que, comme amines aliphatiques tertiaires, on utilise celles répondant à la formule générale (II):

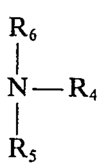

(II)

dans laquelle

$R_4$, $R_5$ et $R_6$ sont identiques ou différents et représentent chacun un groupe alkyl-, cycloalkyl-, N-alcénylaldimino-, N-alcénylcétimino- ou N,N-dialkylaminoalkylène, ou deux ou trois de ces radicaux forment ensemble un système monocyclique ou bicyclique contenant éventuellement un ou plusieurs hétéro-atomes et ayant jusqu'à 10 atomes de carbone.

5. Procédé suivant les revendications 1 et 4, caractérisé en ce qu'on utilise la triméthylamine, la triéthylamine ou la N-méthylpyrrolidine.

6. Procédé suivant la revendication 1, caractérisé en ce que, comme sels d'ammonium quaternaire, on utilise ceux répondant à formule générale (III):

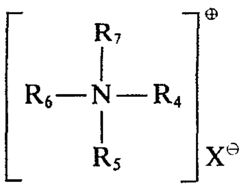

(III)

dans laquelle

$R_4$, $R_5$, $R_6$ et $R_7$ sont identiques ou différents et représentent chacun un groupe alkyl-, cycloalkyl-, N-alcénylaldimino-, N-alcénylcétimino- ou N,N-dialkylaminoalkylène, ou deux ou trois de ces radicaux forment ensemble un système monocyclique ou bicyclique contenant éventuellement un ou plusieurs hétéro-atomes et ayant jusqu'à 10 atomes de carbone, de préférence, jusqu'à 8 atomes de carbone, et

$X^{\ominus}$ représente un chlorure, un bromure, un iodure, un sulfate, un phosphate ou un hydrogénosulfate.

7. Procédé suivant les revendications 1 et 6, caractérisé en ce qu'on utilise le chlorure de tétraméthyl-ammonium ou le chlorure de tétraéthylammonium.

8. Procédé suivant la revendication 1, caractérisé en ce que, comme amidines secondaires, on utilise celles répondant à la formule générale (IV):

$$R_8-NH-CH=N-R_9 \quad (IV)$$

dans laquelle

$R_8$ et $R_9$ sont identiques ou différents et représentent chacun un groupe alkyle ou cycloalkyle ou ensemble, ils forment un système monocyclique ou bicyclique contenant jusqu'à 15 atomes de carbone.

9. Procédé suivant les revendications 1 et 8, caractérisé en ce qu'on utilise l'imidazole.